# EUROPÄISCHE PATENTSCHRIFT

(11) **EP 0 510 446 B1**
(45) Veröffentlichungstag und Bekanntmachung des Hinweises auf die Patenterteilung: **16.12.1998**
(21) Anmeldenummer: 92106142.0
(22) Anmeldetag: 09.04.1992
(51) Int. Cl.: G03F 7/038, G03F 7/004

(54) **Negativ arbeitendes strahlungsempfindliches Gemisch und damit hergestelltes strahlungsempfindliches Aufzeichnungsmaterial**
Negative-working radiation-sensitive composition and radiation-sensitive recording material produced therewith
Composition sensible aux rayonnements travaillant en négatif et matériau d'enregistrement sensible aux rayonnements produit de celle-ci

(30) Priorität: 20.04.1991 DE 4112974
(43) Veröffentlichungstag der Anmeldung: 28.10.1992
(73) Patentinhaber: Clariant GmbH, 65929 Frankfurt am Main (DE)
(72) Erfinder: Röschert, Horst, Dr., W-6531 Ober Hilbersheim (DE); Fuchs, Jürgen, W-6093 Flörsheim/Wicker (DE); Spiess, Walter, Dr., W-6110 Dieburg (DE); Eckes, Charlotte, Dipl.-Ing, W-6200 Wiesbaden-Dotzheim (DE); Pawlowski, Georg, Dr., W-6200 Wiesbaden (DE); Dammel, Ralph, Dr., Coventry, Rhode Island 02816 (US)

(56) Entgegenhaltungen:
- EP-A- 302 019
- EP-A- 388 343
- EP-A- 388 813
- EP-A- 407 086
- EP-A- 444 493
- CHEMICAL ABSTRACTS, vol. 92, no. 24, 16. Juni 1980, Columbus, Ohio, US; abstract no. 198859C, S. PAPPAS ET AL.: 'A new photoinitiator system for radical and cationic polymerization' Seite 6 ;
- PATENT ABSTRACTS OF JAPAN vol. 14, no. 78 (P-1006)(4021) 14. Februar 1990 & JP-A-1 293 339 ( TOSOH CORP. ) 27. November 1989

## Beschreibung

Die Erfindung betrifft ein negativ arbeitendes strahlungsempfindliches Gemisch mit
a) einer Verbindung, die unter Einwirkung von aktinischer Strahlung eine starke Säure bildet,
b) einer Verbindung mit mindestens zwei durch Säure vernetzbaren Gruppen und
c) einem in Wasser unlöslichen, in wäßrig-alkalischen Lösungen löslichen oder zumindest quellbaren polymeren Bindemittel.

Die Erfindung betrifft weiterhin ein damit hergestelltes strahlungsempfindliches Aufzeichnungsmaterial, das zur Herstellung von Photoresists, elektronischen Bauteilen, Druckplatten oder zum Formteilätzen geeignet ist.

Die stetige Verkleinerung der Strukturen, beispielsweise in der Chip-Herstellung bis in den Bereich von weniger als 1 µm, erfordert veränderte lithographische Techniken. Um solche feinen Strukturen abzubilden, verwendet man Strahlung mit einer kurzen Wellenlänge, wie energiereiches UV-Licht, Elektronen- und Röntgenstrahlen. Das strahlungsempfindliche Gemisch muß an die kurzwelliqe Strahlung angepaßt sein. Eine Zusammenstellung der an das strahlungsempfindliche Gemisch gestellten Forderungen ist in dem Aufsatz von C.G. Willson "Organic Resist Materials - Theory and Chemistry" [Introduction to Microlithography, Theory, Materials, and Processing, Herausgeber L.F. Thompson, C.G. Willson, M.J. Bowden, ACS Symp. Ser., 219, 87 (1983), American Chemical Society, Washington] angegeben. Es bestand daher ein verstärkter Bedarf an strahlungsempfindlichen Gemischen, die in den neueren Technologien, wie der Mid- oder Deep-UV-Lithographie, [Belichtung z. B. mit Excimer-Lasern bei Wellenlängen von 305 nm (XeF), 248 nm (KrF), 193 nm (ArF)], der Elektronen- oder der Röntgenstrahl-Lithographie, einsetzbar sind, vorzugsweise darüber hinaus in einem weiten Spektralbereich empfindlich sind und dementsprechend auch in der konventionellen UV-Lithographie verwendet werden können.

Negativ arbeitende strahlungsempfindliche Gemische, die Bisazide als Vernetzer und von Isopren abgeleitete Bindemittel enthalten, sind bekannt. Sie werden als strahlungsempfindliche Schichten bei der Herstellung von Druckplatten, gedruckten Schaltungen und integrierten Schaltkreisen verwendet. Ihr Einsatz in der Mikrolithographie ist jedoch durch verschiedene technische Nachteile begrenzt. So ist es schwierig, qualitativ hochwertige Schichten ohne Fehlstellen (pin-holes) herzustellen. Der Wärmestand solcher Mischungen ist unzureichend, d. h. die Resistbilder werden bei der Verarbeitung durch thermischen Fluß verzerrt. Schließlich ist ihr Auflösungsvermögen auf Strukturen > 2 µm begrenzt, da sie bei der notwendigen Entwicklung mit organischen Lösemitteln auch in den gehärteten Bereichen unerwünscht hohe Quellung zeigen, was wiederum zu Strukturverzerrungen oder inhomogenen Entwicklungsprozessen und damit zu ungenauer Wiedergabe des durch die Belichtungsmaske vorgegebenen Bildes führt. Um Resistbilder mit einer Auflösung von besser als 2 µm herstellen zu können, sind andere negativ arbeitende strahlungsempfindliche Gemische entwickelt worden, die empfindlich gegenüber Strahlung kürzerer Wellenlänge sind, beispielsweise gegenüber energiereicher UV-, Elektronen oder Röntgenstrahlung. Ein derartiges Gemisch enthält beispielsweise ein Copolymer aus (2,3-Epoxy-propyl)-methacrylat und (2,3-Dichlor-propyl)methacrylat (DCOPA) oder eine Kombination der entsprechenden Homopolymeren. Die Glasübergangstemperatur dieses Gemisches ist jedoch für viele Anwendungen zu niedrig, insbesondere ist aber die geringe Plasmaätzbeständigkeit des Gemisches zu bemängeln. Darüber hinaus muß auch dieses Resistmaterial mit Entwicklern auf der Basis von wenig umweltfreundlichen, organischen Lösemitteln verarbeitet werden. Eine geringe Plasmaätzbeständigkeit zeigen auch andere bisher bekannte negativ arbeitende Photoresists auf aliphatischer Basis.

In der EP-A 0 164 248 wurde ein säurehärtbares Gemisch beschrieben, das wäßrig-alkalisch entwickelbar ist, durch die Verwendung von Aromaten eine verbesserte Plasmaätzresistenz aufweist und gegenüber nahem UV-Licht (350 bis 450 nm) empfindlich ist. Als Säurebildner sind dabei insbesondere Sulfonsäureesterderivate des Diazonaphthochinons genannt worden, die bei der Belichtung schwach saure Carbonsäuren bilden und daher nur in vergleichsweise hoher Konzentration wirksam sind. Solche Gemische haben aber infolge der schwachen Absorptionen und des unzureichenden Ausbleichverhaltens des photolytischen Säurebildners eine geringe Empfindlichkeit für DUV-, Elektronen- und Röntgenstrahlung.

In der US-A 3 692 560 wird ein säurehärtbares Gemisch beschrieben, das ein säurevernetzbares Melaminderivat, einen Novolak und chlorierte Benzophenone als photolytische Säurebildner enthält. Auch diese Gemische haben im tiefen UV-Bereich keine ausreichende Empfindlichkeit. Darüber hinaus sind Halogenwasserstoffsäuren als Vernetzungskatalysatoren unerwünscht, da diese bei den nachfolgenden Dotierungsprozessen mit den Dotierungsmitteln Reaktionen eingehen können. Des weiteren wirken im gehärteten Resist verbleibende Halogenwasserstoffsäuren stark korrosiv und können zur Zerstörung des zu bebildernden Materials und der Produktionsgeräte führen.

Das gleiche trifft für die in der EP 0 232 972 erwähnten säurebildenden Derivate des DDT's zu, die hochtoxisch sind und schon von daher nicht praxisgerecht sein können. Immerhin zeigen solche Verbindungen eine beachtliche Empfindlichkeit im tiefen UV-Bereich (200 bis 300 nm).

Als Verbindungen, die bei Bestrahlung eine starke Säure bilden, wurden bisher insbesondere Onium-Salze, wie Diazonium-, Phosphonium-, Sulfonium- und Jodonium-Salze von nicht nucleophilen Säuren, wie HSbF₆, HAsF₆, oder HPF₆ [J.V. Crivello, Polym. Eng. Sci., 23 (1983) 953] verwendet. Daneben sind Halogenverbindungen, insbesondere Trichlormethyltriazin-Derivate oder Trichlormethyloxadiazol-Derivate, o-Chinondiazidsulfonylchloride, o-Chinondiazid-4-sulfonsäureester, Organometall-Organohalogen-Kombinationen, Bis(sulfonyl)diazomethane, Sulfonyl-carbonyl-diazomethane (DE-A 39 30 087) oder Nitrobenzyltosylate [F.M. Houlihan et al., SPIE Proc., Adv. in Resist Techn. and Proc. 920 (1988) 67] empfohlen worden.

Diese Verbindungen werden in negativ oder positiv arbeitenden strahlungsempfindlichen Gemischen verwendet. Die Verwendung derartiger photolytischer Säurebildner bringt aber gewisse Nachteile mit sich, die ihre Einsatzmöglichkeiten in verschiedenen Anwendungsbereichen drastisch einschränken. So sind z.B. viele der Oniumsalze toxisch. Ihre Löslichkeit ist in vielen Lösemitteln unzureichend, weshalb nur wenige Lösemittel zur Herstellung einer Beschichtungslösung geeignet sind. Darüber hinaus werden bei Verwendung der Oniumsalze z.T. unerwünschte Fremdatome eingeführt, die insbesondere in der Mikrolithographie zu Prozeßstörungen führen können. Ferner bilden die Oniumsalze bei der Photolyse sehr stark korrodierend wirkende Brönstedt-Säuren. Diese Säuren greifen empfindliche Substrate an, so daß der Einsatz solcher Gemische zu unbefriedigenden Ergebnissen führt. Wie bereits früher erwähnt, bilden auch die Halogenverbindungen sowie die Chinondiazidsulfonsäurechloride stark korrosiv wirkende Halogenwasserstoffsäuren. Ferner besitzen derartige Verbindungen auf bestimmten Substraten nur eine begrenzte Haltbarkeit, die dadurch verbessert wurde, daß zwischen Substrat und strahlungsempfindlicher, Verbindungen des Typs (a) enthaltender Schicht eine Zwischenschicht eingefügt wurde, was allerdings zu einer unerwünschten Zunahme von Defekten und zu einer verminderten Reproduzierbarkeit führte (DE-A 36 21 376 = US-A 4 840 867).

In neueren Arbeiten von F.M. Houlihan et al., SPIE 920, 67 (1988) wurden anhand positiv arbeitender Systeme gezeigt, daß neben den oben genannten Säurebildnern auch Nitrobenzyltosylate, die bei Belichtung Sulfonsäuren mit geringer Wanderungstendenz bilden, in bestimmten säurelabilen Resistformulierungen verwendbar sind. Es kann aus diesen Ergebnissen abgeleitet werden, daß solche Verbindungen auch für photohärtbare Systeme verwendet werden können. Die dabei erzielten Empfindlichkeiten und die thermische Stabilität der Photoresists erwiesen sich jedoch als unzureichend.

Es ist ferner bekannt, mit Methan-, Ethan-, Propan-, Butan-, Benzol-, Toluol- oder Naphthalinsulfonsäure vollständig verestertes 1,2,3-Trihydroxy-benzol als photoaktiven Säurebildner in positiv arbeitenden Photoresistsystemen zu verwenden [T. Ueno et al., Chemical Amplification Positive Resist Systems Using Novel Sulfonates as Acid Generators, in "Polymers for Microelectronics - Science and Technology", Hrsg. Y. Tabata et al., Kodansha-Weinheim-New York, 1989,S.66-67]. Diese Resistsysteme finden jedoch in der Praxis keine Verwendung, da ihre thermische Stabilität und Plasmaätzresistenz unzureichend ist und nach der Entwicklung Resistreste in den Gräben und nicht akzeptable Resistprofile zu beobachten sind.

In der EP-A 0 388 813 sind negativ-arbeitende, strahlungsempfindliche Gemische beschrieben, die als Säurebildner einen Ester aus einer Alkansulfonsäure und einer Verbindung mit mindestens zwei phenolischen Hydroxygruppen enthalten. Die Alkansulfonsäure umfaßt allgemein nicht mehr als 4 Kohlenstoffatome. Konkret genannt sind Methansulfonsäure, Ethansulfonsäure, Propansulfonsäure, Butansulfonsäure und Trichlormethansulfonsäure.

Es bestand jedoch weiterhin ein Bedarf an Gemischen für negativ-arbeitende, strahlungsempfindliche Aufzeichnungsmaterialien, die eine hohe Empfindlichkeit im DUV-Bereich (200 bis 300 nm) sowie eine hohe Auflösung besitzen, bei Bestrahlung keine korrodierend wirkende Säure freisetzen und wäßrig-alkalisch entwickelbar sind.

Aufgabe der Erfindung war es daher, ein strahlungsempfindliches Gemisch auf Basis von säurebildenden in Kombination mit säurevernetzbaren Verbindungen vorzuschlagen, wobei die photolytisch eine Säure bildende Verbindung (a) möglichst stabil auf allen bekannten Substraten sein sollte und als Photoprodukt eine nicht korrosiv wirkende Säure liefert.

Die Aufgabe wird gelöst durch ein strahlungsempfindliches Gemisch mit
a) einer Verbindung, die unter Einwirkung von aktinischer Strahlung eine starke Säure bildet,
b) einer Verbindung mit mindestens zwei durch Säure vernetzbaren Gruppen und
c) einem in Wasser unlöslichen, in wäßrig-alkalischen Lösungen löslichen oder zumindest quellbaren polymeren Bindemittel,
das dadurch gekennzeichnet ist, daß die Verbindung (a) ein mit 2, 3 oder 4 Sulfonsäuren der allgemeinen Formel R-SO₃H verestertes Di-, Tri- bzw. Tetrahydroxybenzol, das ggf. noch mit einem Rest R' substituiert ist, darstellt, wobei R und R' gleiche oder verschiedene Bedeutung haben und R einen ggf. weiter substituierten (C₅-C₁₀)Cycloalkyl-, (C₆-C₁₀)Aryl-, (C₆-C₁₀)Aryl-(C₁-C₁₀)alkyl- oder (C₃-C₉)Heteroarylrest und R' einen der für R genannten Reste, einen (C₁-C₁₀)Alkyl-; (C₁-C₁₀)Alkanoyl-, (C₁-C₁₆)Alkoxycarbonyl- oder einen (C₇-C₁₁) Aroylrest bezeichnet.

Geeignete Sulfonsäuren R-SO₃H sind beispielsweise Cyclohexan-, Phenylmethan-, 2-Phenyl-ethan-, 3-Phenyl-propan-, Benzol- und Naphthalinsulfonsäure.

Schließlich sind heteroaromatische Sulfonsäuren geeignet. Die Heteroarylsulfonsäuren weisen neben 4 bis 9 Kohlenstoffatomen noch ein aromatisches Sauerstoff- oder Schwefelatom oder 1 oder 2 aromatische Stickstoffatome auf. Beispiele hierfür sind die Furan- und Thiophensulfonsäuren, die Pyrrol-, Pyridin-, Pyrimidin- und Pyrazin-sulfonsäuren. Auch Sulfonsäuren mit einem zweikernigen Heteroarylrest sind geeignet. Hierfür seien die Benzofuran-, Isobenzofuran-, Benzo[b]thiophen- und Indolsulfonsäuren genannt. Die stickstoffhaltigen Heterocyclen dürfen jedoch nicht basisch sein.

Die Reste R und R' können substituiert sein. Im Prinzip können alle Substituenten, die keine unerwünschten Reaktionen eingehen, vorkommen. Geeignete Substituenten sind lineare und verzweigte Alkylgruppen mit vorzugsweise nicht mehr als 8 Kohlenstoffatomen, insbesondere nicht mehr als 6 Kohlenstoffatomen, wie Methyl, Ethyl, Propyl, Isopropyl, Butyl, sec.-Butyl, Isobutyl und tert.-Butyl. Die Alkylgruppen können fluoriert, bevorzugt auch perfluoriert, sein. Von den perfluorierten Alkylresten sind Trifluormethyl und Perfluorbutyl besonders geeignet. Weitere geeignete Substituenten sind (C₁-C₈)Alkoxy, (C₁-C₈)Alkanoyl, -(C₁-C₈)alkoxy, (C₁-C₈) Alkanoyl, (C₁-C₈)-Alkanoyloxy, (C₁-C₈)Alkanoylamino, (C₆-C₁₀)Aryl, (C₆-C₁₀)Aryloxy, (C₆-C₁₀)Aryl-(C₁-C₈)alkoxy, (C₆-C₁₁)-Aroylamino, (C₆-C₁₁)Aroylamino-(C₁-C₆)alkyl, Cyano und Halogen. Die Substituenten können mehrfach auftreten. Unabhängig davon können verschiedene Substituenten nebeneinander vorhanden sein. Bevorzugte Substituenten sind (C₁-C₄)Alkyl, (C₁-C₄)Alkoxy und Phenyl. Der Phenylrest ist wiederum bevorzugt mit (C₁-C₆)Alkyl, (C₁-C₈)Perfluoralkyl und/oder Halogen, insbesondere Fluor, substituiert. Bevorzugt steht einer dieser Substituenten in der para-Position.

Der ggf. vorhandene Substituent R' ist insbesondere Methyl, Ethyl, Propyl, Isopropyl, Butyl, sec.-Butyl, Isobutyl, tert.-Butyl, Formyl, Acetyl, Methoxycarbonyl, Phenyl, Benzyl, Cyclohexyl, Benzoyl, Phenethyl, 3-Phenyl-propyl, [1]Naphthyl oder [2]Naphthyl. Die Heteroarylreste weisen neben 4 bis 9 Kohlenstoffatomen noch ein aromatisches Sauerstoff- oder Schwefelatom oder 1 oder 2 Stickstoffatome auf. Beispiele sind Furanyl-, Thiophenyl-, Pyrrolyl-, Pyridinyl- und Chinolinylreste, wobei die stickstoffhaltigen Heterocyclen jedoch nicht basisch sein dürfen. Die Reste R' können auf die gleiche Weise substituiert sein wie die Reste R. Sie können darüber hinaus auch mit Hydroxy und Sulfonyloxy (O-SO₂-R, wobei R die obengenannte Bedeutung hat) substituiert sein. Die aromatischen Reste R' sind bevorzugt mit (C₁-C₄)Alkoxy, (C₁-C₈)Alkanoyl, Sulfonyloxy (-O-SO₂-R) und Halogen substituiert. Falls mehrere Substituenten an einen aromatischen Rest gebunden sind, so sind diese bevorzugt ausgewählt aus der Gruppe bestehend aus (C₁-C₄)Alkyl, Hydroxy, Sulfonyloxy (-O-SO₂-R) und Halogen.

Neben Di-, Tri- und Tetrahydroxybenzolen sind auch Di-, Tri- und Tetrahydroxyphenyl-Reste enthaltende Polymere als Ausgangsmaterial zur Herstellung der mehrfunktionellen Sulfonsäureester geeignet. In solchen Polymeren kommen beispielsweise Einheiten abgeleitet aus (Meth)acrylsäure-dihydroxyphenylester vor. Einheiten ohne Dihydroxyphenylrest können neben Einheiten mit diesem Rest vorliegen, d. h. neben Homopolymeren sind auch Co- und Terpolymere sowie Polymere noch mehr verschiedenen Einheiten geeignet. Die weiteren Einheiten leiten sich bevorzugt von Styrol, einem Hydroxystyrol, Maleimid, einem N-substituierten Maleimid, einem Vinyl-alkyl-ether und/oder einem Vinyl-alkyl-silan ab. Homo- und Copolymere, insbesondere Copolymere mit Maleimid oder Styrol sind bevorzugt. Kondensationsprodukte aus einem Trihydroxybenzol, wie 1,2,3- und 1,2,4-Trihydroxy-benzol, mit einem Keton sind als Ausgangsmaterialien gleichfalls geeignet. Mehrfunktionelle Sulfonsäureester aus polymeren Ausgangsmaterialien sind jedoch nicht bevorzugt.

Bevorzugte Sulfonsäuren sind Cyclohexan-, Phenylmethan-, 2-Phenylethan-, 3-Phenyl-propan-, Benzol-, 3-Perfluoroctylbenzol-, 4-Trifluormethylbenzol-, 4-Perfluorbutylbenzol-, 4-Tolyl-, 4-Brombenzol-, 4-Cyanobenzol-, 4-tert.-Butylbenzol-, 2,4,5-Trimethylbenzol-, 3,4-Dichlorbenzol-, (+)Campher-10- und 2-Benzoylaminomethyl-thiophen-5-sulfonsäure.

Besonders geeignete Ausgangsverbindungen mit phenolischen Hydroxygruppen sind:

Brenzkatechin, Resorcin, Hydrochinon, 2,4-Dihydroxybenzaldehyd, 3,4-Dihydroxy-benzaldehyd, 3,5-Dihydroxybenzaldehyd, Poly[methacrylsäure-(2,3-dihydroxy-phenyl)-ester], Poly[methacrylsäure-(3,5-dihydroxy-phenyl)-ester], Poly[methacrylsäure-(2,4-dihydroxyphenyl)-ester], 2,4,2',4'-Tetrahydroxybenzophenon, 2,4,4'-Tri-hydroxybenzophenon, 2,4-Dihydroxybenzophenon, Pyrogallol, Phloroglucin, 1,2,4-Trihydroxy-benzol, 2,3,4-Tri-hydroxy-benzaldehyd, Gallusaldehyd, Gallussäuremethylester, Gallussäure-propylester, Gallussäure-octylester, Gallussäure-dodecylester, 2,3,4-Trihydroxybenzophenon, 2,4,6-Trihydroxybenzophenon, 2,3,4-Trihydroxyacetophenon, 2,4,6-Trihydroxyacetophenon, 1-Octanoyl-2,3,4-trihydroxy-benzol, 1-Hexanoyl-2,3,4-trihydroxy-benzol, 1-Butyryl-2,3,4-trihydroxy-benzol, 1-Octanoyl-2,4,6-trihydroxy-benzol, 1-Hexanoyl-2,4,6-trihydroxy-benzol, 1-Butyryl-2,4,6-trihydroxy-benzol sowie Kondensationsprodukte aus Pyrogallol und Ketonen. Di- und Trihydroxy-benzole bzw. Polymere mit Di- und Trihydroxyphenylgruppen sind allgemein bevorzugt. Nicht vollständig veresterte Produkte, d. h. solche, die noch freie phenolische Hydroxygruppen aufweisen, sind in speziellen strahlungsempfindlichen Gemischen besser löslich.

Die Eignung der erfindungsgemäßen Sulfonsäureester in negativ arbeitenden Gemischen war nicht naheliegend, da sie bei Bestrahlung lösliche Spaltprodukte (Phenole und Sulfonsäuren) in den zu vernetzenden Bereichen liefern.

Die Herstellung der in dem erfindungsgemäßen Gemisch eingesetzten, mehrfunktionellen Sulfonsäureester ist an sich bekannt. Als Ausgangsmaterialien dienen dabei in erster Linie die entsprechenden Sulfonsäurechloride. Verfahren zu Herstellung von aromatischen Sulfonsäureestern werden beispielsweise von F. Muth in: Houben-Weyl-Müller, Methoden der organischen Chemie, Bd. IX, S. 633 (und dort zitierte Literatur), Thieme-Verlag, 4. Aufl., Stuttgart 1955, und von S. Pawlenko, a.a.O., Bd. E 11, S. 1084, Thieme-Verlag, 1. Aufl., Stuttgart 1985, sowie in der Patentliteratur an zahlreichen Beispielen beschrieben. Auch die entsprechenden Sulfonsäureanhydride sind als Ausgangsmaterialien geeignet (siehe S. Pawlenko, a.a.O., Bd. Ell, S. 1086, Thieme-Verlag, 1. Aufl., stuttgart 1985, und P. J. Stang, M. Hanack, L. R. Subramaniam, Synthesis, **1982**, 85). Dies gilt insbesondere für die mit Perfluoralkyl-Gruppen substituierten Benzolsulfonsäureanhydride.

Die mehrfunktionellen Sulfonsäureester, in denen R einen perfluoralkylsubstituierten Phenylrest darstellt, wurden im Gegensatz zu den übrigen beschriebenen Sulfonsäureestern durch Umsetzung der entsprechenden Sulfonsäurefluoride mit den Trimethylsilanyloxy-benzolen nach dem von H. Niederprüm, V. Beyl, P. Voss (Liebigs Ann. Chem. **1973**, 20) angegebenen Verfahren hergestellt. Die Trimethylsilanyloxy-Derivate der Di-, Tri- und Tetrahydroxybenzole werden durch Umsetzung mit Hexamethyldisilazan analog den in den Monographien von E. W. Colvin, Silicon Reagents in Organic Synthesis, 1. Aufl., S. 91 - 97, Academic Press, London 1988, und T. W. Greene, Protective Groups in Organic Synthesis, 1. Aufl., S. 100, J. Wiley & Sons, New York 1981, beschriebenen Verfahren erhalten.

Das erfindungsgemäße, strahlungsempfindliche Gemisch zeichnet sich durch eine hohe Empfindlichkeit über einen weiten Spektralbereich aus. Es zeigt eine hohe thermische Stabilität und schafft die Möglichkeit, auch feinste Strukturen einer Vorlage detailgenau wiederzugeben. Die bei der Bestrahlung gebildete Säure wirkt nicht korrodierend, so daß das Gemisch auch auf empfindlichen Substratmaterialien Verwendung finden kann.

Überraschenderweise zeigen die erfindungsgemäßen, negativ arbeitenden, strahlungsempfindlichen Gemische nicht nur eine hohe thermische und Plasmaätzbeständigkeit sondern auch hervorragende lithographische Eigenschaften, die eine Auflösung im Halbmikrometer- und teilweise auch im Sub-Halbmikrometer-Bereich erlauben. Man erhält nach dem bildmäßigen Bestrahlen und anschließendem Entwickeln ein detailgetreues Abbild der Maske. Die Resistfelder weisen steile Flanken auf. In den nichtbestrahlten Bereichen wird die Resistschicht vollständig abgelöst, d. h. es bleiben keinerlei Reste oder Rückstände der Schicht auf dem Substrat. Die bei der Photolyse gebildeten Sulfonsäuren führen zu einer effizienten Vernetzung der Resist-Komponenten b) und c), was die Herstellung von hochempfindlichen, negativ arbeitenden Gemischen erlaubt.

Mit den erfindungsgemäßen Gemischen hergestellte Aufzeichnungsmaterialien zeigen überraschend eine höchsten Ansprüchen genügende Bilddifferenzierung und, noch überraschender, eine Verbesserung des Kontrasts und des Auflösungsvermögens. Die erfindungsgemäßen Gemische erlauben beispielsweise die Herstellung eines hochempfindlichen negativ arbeitenden Photoresists für energiereiche UV2-Strahlung (z. B. 248 nm).

Da das erfindungsgemäße Gemisch über einen weiten Spektralbereich empfindlich ist, ist zum bildmäßigen Bestrahlen aktinische Strahlung allgemein geeignet. Als aktinische Strahlung soll in diesem Zusammenhang jede Strahlung verstanden werden, deren Energie mindestens der des kurzwelligen sichtbaren Lichts entspricht. Geeignet ist dabei insbesondere UV-Strahlung im Bereich von 190 bis 450 nm, bevorzugt von 200 bis 400 nm, besonders bevorzugt von 200 bis 300 nm, aber auch Elektronenoder Röntgenstrahlung.

Die im erfindungsgemäßen, strahlungsempfindlichen Gemisch enthaltenen, bei Bestrahlung Säure bildenden mehrfunktionellen Sulfonsäureester können allein oder in Kombination mit anderen Säurebildnern Verwendung finden. Als zusätzliche Säurebildner sind insbesondere die in der gleichzeitig eingereichten deutschen Patentanmeldung P 41 12 972.5 beschriebenen mehrfunktionellen Sulfonsäureester des 2,4,6-Tris-(2-hydroxy-ethoxy)-[1,3,5]triazins geeignet.

Darüber hinaus lassen sich die mehrfunktionellen Sulfonsäureester auch mit Onium-Salzen, Halogenverbindungen, insbesondere Trichlormethyltriazinderivaten oder Trichlormethyloxadiazolderivaten, 1,2-Disulfonen, o-Chinondiazidsulfonylchloriden oder Organometall-Organohalogen-Kombinationen kombinieren. Es kommen aber auch Mischungen mit Bis(sulfonyl)diazomethanen und Sulfonyl-carbonyldiazomethanen in Frage. In solchen Gemischen können jedoch die eingangs erwähnten Nachteile wieder auftreten.

Der Anteil an mehrfunktionellen Sulfonsäureestern im erfindungsgemäßen Gemisch liegt im allgemeinen bei 0,5 bis 25 Gew.-%, bevorzugt bei 3 bis 15 Gew.-%, bezogen auf das Gesamtgewicht der Feststoffe im Gemisch.

Als säurevernetzbare Verbindungen b) kommen besonders die in der GB 2 082 339 offenbarten Resole, daneben auch mit Alkoxymethyl- oder Oxiranylmethyl-Gruppen substituierte Aromaten (EP-A 0 212 482) sowie monomere und oligomere Melamin- bzw. Harnstoff-Formaldehyd-Kondensate (EP-A 0 133 216, DE-A 36 34 371, DE 37 11 264) in Betracht. Beispiele für den ersten Typ sind insbesondere die kommerziell erhältlichen Resolprodukte Bakelite R 5363, Bakelite R 17620, Bakelite R 10282 und Kelrez 40-152 (Bakelite und Kelrez sind eingetragene Warenzeichen). Resolderivate sind jedoch insgesamt nicht bevorzugt, da sie im tiefen UV-Bereich verhältnismäßig hohe Absorptionen aufweisen und damit zu einer Beeinträchtigung der Bildwiedergabe führen.

Besser geeignet sind die aus der EP-A 0 212 482 bekannten Vernetzer der allgemeinen Formel **I**

(R¹O-CHR³)ₙ-A-(CHR³-OR²)ₘ **(I)**

in der
- A: -B- oder -B-Y-B- ist und
- B: einen ggf. substituierten einkernigen aromatischen Kohlenwasserstoff oder eine Sauerstoff oder Schwefel enthaltende heterocyclische aromatische Verbindung darstellt,
- Y: eine Einfachbindung, (C₁-C₄)Alkylen oder (C₁-C₄)Alkylendioxy, deren Ketten durch Sauerstoffatome unterbrochen sein können, -O-, -S-, -SO₂-, -CO-, -CO₂-, -O-CO₂-, -CONH- oder -O-C₆H₄-O- bedeutet,
- R¹ und R²: Wasserstoff, (C₁-C₆)Alkyl, C₅ oder C₆-Cycloalkyl-, ggf. substituiertes (C₆-C₁₂)Aryl, (C₆-C₁₂)Aralkyl oder Acyl ist,
- R³: Wasserstoff, (C₁-C₄)Alkyl oder ggf. substituiertes Phenyl,
- n: eine ganze Zahl von 1 bis 3 und
- m: eine ganze Zahl von 0 bis 3, wobei n+m mindestens 2 ist, bedeuten.

Typische Vernetzer sind demnach Aromaten und Heterocyclen, die mehrfach mit Hydroxymethyl-, Acetoxymethyl- und Methoxymethyl-Gruppen substituiert sind.

Weitere bevorzugte Vernetzer sind Melamin/Formaldehyd-Derivate, die beispielsweise mindestens zwei freie N-Hydroxymethyl-, N-Alkoxymethyl- oder N-Acyloxymethylgruppen aufweisen. Insbesondere die N-Alkoxymethylderivate sind zum Einsatz in dem erfindungsgemäßen strahlungsempfindlichen Gemisch geeignet.

Die Vernetzer sind in Lage, bei erhöhten Temperaturen unter dem Einfluß der photolytisch erzeugten Säure mit den polymeren Bindemitteln zu vernetzen. Allgemein sind sie dadurch gekennzeichnet, daß sie unter den genannten Temperatur- und Säurebedingungen ein Carbonium-ion bilden können.

Der Anteil der säurevernetzbarem Verbindung b) am erfindungsgemäßen strahlungsempfindlichen Gemisch liegt zweckmäßig bei 1 bis 50 Gew.-%, vorzugsweise bei 5 bis 25 Gew.-%, jeweils bezogen auf das Gesamtgewicht der festen Bestandteile des Gemisches.

Das erfindungsgemäße strahlungsempfindliche Gemisch enthält ferner mindestens ein polymeres, in Wasser unlösliches, in wäßrig-alkalischen Lösungen dagegen lösliches, zumindest quellbares Bindemittel c). Das Bindemittel zeichnet sich im besonderen dadurch aus, daß es mit den übrigen Bestandteilen des erfindungsgemäßen strahlungsempfindlichen Gemisches gut verträglich ist und insbesondere im Wellenlängenbereich von 190 bis 300 nm eine möglichst geringe Eigenabsorption, d.h. eine hohe Transparenz, aufweist. Bindemittel auf der Basis von Novolak-Kondensationsharzen, die in der Regel in Kombination mit Naphthochinondiaziden als photoaktive Komponenten eingesetzt werden, erfüllen diese Bedingung nicht. Zwar lassen Novolak-Kondensationsharze nach bildmäßiger Belichtung in den nicht belichteten Bereichen eine Erniedrigung der Löslichkeit gegenüber wäßrig-alkalischen Entwicklern erkennen, doch ist ihre Eigenabsorption im Bereich der für die Bestrahlung gewünschten kurzen Wellenlänge unerwünscht hoch.

Novolak-Kondensationsharze können aber in Mischung mit anderen als Bindemittel geeigneten Harzen mit höherer Transparenz eingesetzt werden. Die Mischungsverhältnisse richten sich dabei vorwiegend nach der Art des mit dem Novolakharz zu mischenden Bindemittels. Insbesondere spielen dessen Grad an Eigenabsorption im genannten Wellenlängenbereich, aber auch die Mischbarkeit mit den anderen Bestandteilen des strahlungsempfindlichen Gemisches eine entscheidende Rolle. Im allgemeinen kann aber das Bindemittel des erfindungsgemäßen strahlungsempfindlichen Gemisches bis zu 30 Gew.-%, insbesondere bis zu 20 Gew.-%, eines Novolak-Kondensationsharzes enthalten.

Das Bindemittel(gemisch) weist vorteilhaft eine Extinktion für Strahlung der Wellenlänge 248 nm von < 0,5 µm⁻¹, bevorzugt < 0,3 µm⁻¹, auf.

Als Bindemittel geeignet sind Homo- oder Copolymere des p-Hydroxystyrols sowie seiner Alkylderivate, z.B. des 3-Methyl-4-hydroxystyrols, sowie Homo- oder Copolymere anderer Vinylphenole, z. B. des 3-Hydroxystyrols oder der Ester oder Amide von Acrylsäure mit phenolischen Gruppen aufweisenden Aromaten. Als Comonomere können polymerisierbare Verbindungen wie Styrol, Methyl(meth)acrylat oder ähnliche eingesetzt werden.

Gemische mit erhöhter Plasmabeständigkeit werden erhalten, wenn zur Herstellung der Bindemittel Silicium enthaltende Vinylmonomere, z. B. Vinyltrimethylsilan, mitverwendet werden. Die Transparenz dieser Bindemittel ist im interessierenden Bereich im allgemeinen höher, so daß eine verbesserte Strukturierung möglich ist.

Mit gleichem Erfolg lassen sich auch Homo- oder Copolymere des Maleimids verwenden. Auch diese Bindemittel zeigen hohe Transparenz im beschriebenen Wellenlängenbereich. Als Comonomere werden auch hier bevorzugt Styrol, substituierte Styrole, Vinylether, Vinylester, Vinylsilylverbindungen oder (Meth)acrylsäureester eingesetzt.

Schließlich sind darüber hinaus auch Copolymere des Styrols mit Comonomeren verwendbar, die in wäßrig alkalischen Lösungen eine Löslichkeitserhöhung bewirken. Hierzu zählen beispielsweise Maleinsäureanhydrid und Maleinsäurehalbester.

Die genannten Bindemittel können gemischt werden, sofern sich die optische Qualität des strahlungsempfindlichen Gemisches dadurch nicht verschlechtert. Bindemittelgemische sind jedoch nicht bevorzugt.

Der Anteil des Bindemittels beträgt im allgemeinen 40 bis 95 Gew.-%, insbesondere 50 bis 90 Gew.-%, bezogen auf das Gesamtgewicht der festen Anteile des strahlungsempfindlichen Gemisches.

Ferner können den erfindungsgemäßen strahlungsempfindlichen Gemischen gegebenenfalls Farbstoffe, Pigmente, Weichmacher, Netzmittel und Verlaufmittel, aber auch Polyglykole, Celluloseether, z. B. Ethylcellulose, zur Erfüllung spezieller Erfordernisse, wie Flexibilität, Haftung und Glanz, zugesetzt werden.

Soll ein Substrat beschichtet werden, so wird das erfindungsgemäße strahlungsempfindliche Gemisch zweckmäßig in einem Lösemittel oder in einer Kombination von Lösemitteln gelöst. Hierfür besonders geeignet sind Ethylenglykol und Propylenglykol sowie die davon abgeleiteten Mono- und Dialkylether, besonders die Mono- und Dimethylether sowie die Mono- und Diethylether, Ester abgeleitet aus aliphatischen (C₁-C₆)Carbonsäuren und entweder (C₁-C₈)Alkanolen oder (C₁-C₈)Alkandiolen oder (C₁-C₆)Alkoxy-(C₁-C₈)alkanolen, beispielsweise Ethylacetat, Hydroxyethylacetat, Alkoxyethylacetat, n-Butylacetat, Propylenglykolmonoalkyletheracetat, insbesondere Propylenglykolmethyletheracetat und Amylacetat, Ether, wie Tetrahydrofuran und Dioxan, Ketone, wie Methylethylketon, Methylisobutylketon, Cyclopentanon und Cyclohexanon, N,N-Dialkyl-carbonsäureamide, wie N,N-Dimethylformamid und N,N-Dimethylacetamid, aber auch Hexamethylphosphorsäuretriamid, N-Methyl-pyrrolidin-2-on und Butyrolacton, sowie beliebige Mischungen davon. Besonders bevorzugt von diesen sind die Glykolether, aliphatischen Ester und Ketone.

Letztendlich hängt die Wahl des Lösemittels bzw. Lösemittelgemisches ab von dem angewandten Beschichtungsverfahren, der gewünschten Schichtstärke und den Trocknungsbedingungen. Ebenso müssen die Lösemittel unter den angewandten Bedingungen gegenüber den übrigen Schichtbestandteilen chemisch inert sein.

Die mit den genannten Lösemitteln hergestellte Lösung hat in der Regel einen Feststoffgehalt von 5 bis 60 Gew.-%, vorzugsweise bis 50 Gew.-%.

Gegenstand der Erfindung ist schließlich auch ein strahlungsempfindliches Aufzeichnungsmaterial, das im wesentlichen aus einem Substrat und einer darauf befindlichen strahlungsempfindlichen Schicht, die das erfindungsgemäße Gemisch enthält, besteht.

Als Substrate kommen alle Materialien in Frage, aus denen Kondensatoren, Halbleiter, mehrlagige gedruckte Schaltungen oder integrierte Schaltkreise bestehen bzw. hergestellt werden können. Speziell sind Siliciumsubstrate zu nennen, die auch thermisch oxidiert und/oder mit Aluminium beschichtet, aber auch dotiert sein können. Daneben sind alle anderen in der Halbleitertechnologie üblichen Substrate möglich, wie Siliciumnitrid, Galliumarsenid und Indiumphosphid. Weiterhin kommen die aus der Flüssigkristalldisplay-Herstellung bekannten Substrate in Frage, wie z. B. Glas und Indium-Zinnoxid, ferner Metallplatten und -folien - beispielsweise aus Aluminium, Kupfer, Zink-, Bimetall- und Trimetallfolien, aber auch elektrisch nichtleitende Folien, die mit Metallen bedampft sind, und Papier. Diese Substrate können thermisch vorbehandelt, oberflächlich aufgerauht, angeätzt oder, zur Verbesserung erwünschter Eigenschaften, z. B. zur Erhöhung der Hydrophilie, mit Chemikalien vorbehandelt sein.

Um der strahlungsempfindlichen Schicht einen besseren Zusammenhalt und/oder eine bessere Haftung auf der Substratoberfläche zu verleihen, kann in ihr ein Haftvermittler enthalten sein. Bei Silicium- bzw. Siliciumdioxid-Substraten kommen hierfür Haftvermittler vom Aminosilan-Typ, wie z. B. 3-Aminopropyltriethoxysilan oder Hexamethyldisilazan, in Frage.

Geeignete Träger für die Herstellung von photomechanischen Aufzeichnungsschichten, wie Druckformen für den Hochdruck, Flachdruck, Siebdruck und Flexodruck sind besonders Aluminiumplatten, die gegebenenfalls vorher anodisch oxidiert, gekörnt und/oder silikatisiert werden, daneben Zink- und Stahlplatten, die gegebenenfalls verchromt werden, sowie Kunststoffolien und Papier.

Das erfindungsgemäße Aufzeichnungsmaterial wird mit aktinischer Strahlung bildmäßig belichtet. Als Strahlungsquellen eignen sich besonders Metallhalogenidlampen, Kohlebogenlampen, Xenonlampen und Quecksilberdampflampen. Ebenso kann eine Belichtung mit energiereicher Strahlung wie Laser-, Elektronen- oder Röntgenstrahlung erfolgen. Besonders bevorzugt sind jedoch Lampen, die Licht einer Wellenlänge von 190 bis 260 nm ausstrahlen können, d.h. insbesondere Xenon- und Quecksilberdampflampen. Darüber hinaus lassen sich auch Laserlichtquellen verwenden, z. B. Excimerlaser, insbesondere KrF- oder ArF-Laser, die bei 248 bzw. 193 nm emittieren. Die Strahlungsquellen müssen in den genannten Wellenlängenbereichen eine ausreichende Emission aufweisen.

Die Stärke der lichtempfindlichen Schicht hängt vom Verwendungszweck ab. Sie beträgt im allgemeinen zwischen 0,1 und 100 µm, bevorzugt zwischen 0,5 und 10 µm, besonders bevorzugt um 1,0 µm.

Gegenstand der Erfindung ist ferner ein Verfahren zur Herstellung eines strahlungsempfindlichen Aufzeichnungsmaterials. Das Auftragen des strahlungsempfindlichen Gemisches auf das Substrat kann durch Aufsprühen, Fließbeschichten, Walzen, Schleuder- und Tauchbeschichten erfolgen. Danach wird das Lösemittel durch Verdampfen entfernt, so daß auf der Oberfläche des Substrats die strahlungsempfindliche Schicht zurückbleibt. Die Entfernung des Lösemittels kann durch Erhitzen der Schicht auf Temperaturen bis zu 150 °C gefördert werden. Das Gemisch kann aber auch zunächst auf obengenannte Weise auf einen Zwischenträger aufgetragen werden, von dem aus es unter Druck und erhöhter Temperatur auf das endgültige Trägermaterial übertragen wird. Als Zwischenträger können grundsätzlich alle auch als Trägermaterialien geeigneten Materialien Anwendung finden. Anschließend wird die Schicht bildmäßig bestrahlt. Danach behandelt man die Schicht mit einer Entwicklerlösung, die die nicht bestrahlten Bereiche der Schicht löst und entfernt, so daß ein Abbild der bei der bildmäßigen Bestrahlung verwendeten Vorlage auf der Substratoberfläche verbleibt.

Als Entwickler eignen sich besonders wäßrige Lösungen, die Silikate, Metasilikate, Hydroxide, Hydrogen- und Dihydrogenphosphate, Carbonate oder Hydrogencarbonate von Alkalimetall-, Erdalkalimetall- und/oder Ammoniumionen enthalten, aber auch Ammoniak und dergleichen. Metallionenfreie Entwickler werden in der US-A 4 729 941, der EP-A 0 062 733, der US-A 4 628 023, der US-A 4 141 733, der EP-A 0 097 282 und der EP-A 0 023 758 beschrieben. Der Gehalt dieser Substanzen in der Entwicklerlösung beträgt im allgemeinen 0,1 bis 15 Gew.-%, vorzugweise 0,5 bis 5 Gew.-%, bezogen auf das Gewicht der Entwicklerlösung. Metallionenfreie Entwickler werden bevorzugt verwendet. Den Entwicklern können gegebenenfalls geringe Mengen eines Netzmittels zugesetzt sein, um die Ablösung der löslichen Bereiche der Schicht zu erleichtern.

Die entwickelten Schichtstrukturen können nachgehärtet werden. Dies geschieht im allgemeinen durch Erhitzen auf einer hot-plate bis zu einer Temperatur unterhalb der Fließtemperatur und anschließendes ganzflächiges Belichten mit dem UV-Licht einer Xenon-Quecksilber-Dampflampe (Bereich von 200 bis 250 nm). Durch die Nachhärtung werden die Bildstrukturen vernetzt, so daß sie im allgemeinen eine Fließbeständigkeit bis zu Temperaturen von über 200 °C aufweisen. Die Nachhärtung kann auch ohne Temperaturerhöhung allein durch Bestrahlen mit energiereichem UV-Licht erfolgen.

Verwendung finden die erfindungsgemäßen Verbindungen in strahlungsempfindlichen Gemischen zur Herstellung integrierter Schaltungen oder von diskreten elektrischen Bausteinen in lithographischen Prozessen, da sie eine hohe Lichtempfindlichkeit aufweisen, besonders bei Bestrahlung mit Licht einer Wellenlänge zwischen 190 bis 300 nm. Da die Gemische bei Belichtung sehr gut ausbleichen, läßt sich eine sehr hohe Auflösung erzielen, die mit den bekannten Gemischen nicht erreicht wird. Das aus dem Gemisch hergestellte Aufzeichnungsmaterial dient als Maske für die folgenden Prozeßschritte. Hierunter zählen z. B. das Ätzen des Schichtträgers, das Implantieren von Ionen in den Schichtträger oder das Abscheiden von Metallen oder anderen Materialien auf den Schichtträger.

Die nachstehend beschriebenen Beispiele illustrieren die Erfindung, ohne sie zu beschränken.

### Synthesebeispiel

### 1,3,5-Tris-methansulfonyloxy-benzol

Zu einer Lösung von 12,6 g (0,10 mol) Phloroglucin und 101 g (1,0 mol) N-Methylmorpholin in 200 ml trokkenem Tetrahydrofuran wurden bei 0 °C 39,8 g (0,33 mol) Methansulfonsäurechlorid innerhalb von 30 min so zugetropft, daß die Temperatur nicht über 10 °C anstieg. Nach dem Zutropfen wurde das Eisbad entfernt, auf Raumtemperatur erwärmt und 2 h bei dieser Temperatur gerührt. Die Mischung wurde nun unter ständigem Rühren in 3 1 Eiswasser langsam eingetropft, der entstandene Niederschlag abgesaugt und mit Wasser neutral gewaschen. Nach dem Trocknen wurden 32,9 g (91 %) eines farbloses Pulvers mit Schmelzpunkt von 145 bis 149 °C erhalten. Die Umkristallisation aus einem Lösemittelgemisch Isopropanol/Aceton ergab farblose, stark lichtbrechende Plättchen mit einem Schmelzpunkt von 150 °C.

Die Analyse dieser Verbindung ergab folgende Werte:

| | | | |
|---|---|---|---|
| ber.: | C 30,00 % | H 3,36 % | S 26,69 % |
| gef.: | C 29,8 % | H 3,2 % | S 26,5 % |

Die Charakterisierung der mehrfunktionellen Sulfonsäureester erfolgte durch ¹H-und ¹³C-Hochfeld-Kernresonanzspektren sowie durch Elementaranalysen und ggf. durch IR-Spektren zum Beweis der Abwesenheit von Hydroxygruppen im Produkt.

Die Beispiele 1 bis 8 belegen die Eignung des erfindungsgemäßen Gemisches für Aufzeichnungsmaterialien in der Mikrolithographie unter Verwendung unterschiedlichster Energie. Anhand des Vergleichsbeispiels 9 und 10 wird die Überlegenheit der erfindungsgemäßen Gemische gegenüber dem Stand der Technik belegt. Die Beispiele 11 und 12 dokumentieren die Anwendbarkeit des Gemisches in gedruckten Schaltungen und Flachdruckplatten.

### Anwendungsbeispiele

Die Beschichtungslösungen wurden durch Filter mit einem Porendurchmesser von 0,2 µm filtriert und auf einen mit einem Haftvermittler (Hexamethyldisilazan) vorbehandelten Wafer aufgeschleudert. Die Schleuderdrehzahl wurde dabei so gewählt, daß man nach 1 min Trocknen bei 110 °C auf der hot plate Schichtdicken um 1,07 µm erhielt.

Sofern bei den einzelnen Beispielen nichts anderes angegeben ist, wurde das Aufzeichnungsmaterial bildmäßig unter einer Vorlage mit der Strahlung eines KrF-Excimerlasers (248 nm) oder einer Xenon-Quecksilberdampflampe (260 nm, mit Interferenzfilter) belichtet und anschließend 1 min bei 110 °C einem post exposure bake auf einer hot plate unterzogen.

Entwickelt wurde das Aufzeichnungsmaterial mit einer 0,27 n wäßrigen Tetramethylammoniumhydroxid-Lösung.

In den folgenden Beispielen wurden Gewichtsteile mit Gt abgekürzt.

### Beispiel 1:

Es wurde ein lichtempfindliches Aufzeichnungsmaterial hergestellt mit Hilfe einer Beschichtungslösung, bestehend aus

| | |
|---|---|
| 7,5 Gt | eines Copolymeren aus Styrol und Maleimid (Molverhältnis 50:50) mit einem Erweichungsbereich von 165 bis 180 °C, |
| 2,0 Gt | Hexa-N-acetoxymethyl-melamin und |
| 0,4 Gt | 1-Octanoyl-2,3,4-tris-(toluol-4-sulfonyloxy)benzol (hergestellt analog dem Synthesebeispiel) in |
| 42 Gt | Propylenglykol-monomethylether-acetat. |
| | |
| Belichtung | 36 mJ/cm² (Xenon-Quecksilberdampflampe) |
| Post exposure bake | 2 min, 120 °C, hot plate |
| Entwicklung | 75 s (0,02 n wäßrige Tetramethylammoniumhydroxid-Lösung) |

### Beispiel 2:

Es wurde ein lichtempfindliches Aufzeichnungsmaterial hergestellt mit Hilfe einer Beschichtungslösung, bestehend aus

| | |
|---|---|
| 7,5 Gt | des in Beispiel 3 genannten Copolymeren, |
| 2,5 Gt | 4,4'-Bis-methoxymethyl-diphenylether und |
| 0,9 Gt | 1,2,3-Tris(4-brom-benzolsulfonyloxy)-benzol (hergestellt analog dem Synthesebeispiel) in |
| 42 Gt | Propylenglykol-monomethylether-acetat. |
| | |
| Belichtung: | 46 mJ/cm² (Xenon-Quecksilberdampflampe) |
| Post exposure bake: | 2 min, 115 °C, hot plate |
| Entwicklung: | 75 s (0,02 n wäßrige Tetramethylammoniumhydroxid-Lösung) |

### Bewertung der entwickelten Aufzeichnungsmaterialien

Die gemäß den Beispielen 1 und 2 erhaltenen Resiststrukturen stellten ein fehlerfreies, negatives Abbild der Maske mit steilen Resistflanken dar, wobei Strukturen bis 0,50 um und teilweise auch darunter detailgetreu wiedergegeben waren.

Eine rasterelektronenmikroskopische Untersuchung zeigte, daß die Resistflanken senkrecht zur Substratoberfläche ausgerichtet waren. Die Schichtabträge in den belichteten Resistbereichen lagen in allen Fällen bei etwa 20 nm/min oder darunter.

### Beispiele 3 und 4 (Vergleichsbeispiele):

Es wurde ein lichtempfindliches Aufzeichnungsmaterial hergestellt mit Hilfe einer Beschichtungslösung, bestehend aus

| | |
|---|---|
| 7,5 Gt | eines Homopolymeren aus 3-Methyl-4-hydroxystyrol mit einem Erweichungsbereich von ≥ 150 °C, |
| 2,0 Gt | Hexa-N-methoxymethyl-melamin und |
| 0,8 Gt | Triphenylsulfonium-hexafluorophosphat (Beispiel 3) bzw. 2-Nitro-benzyltosylat (Beispiel 4) in |
| 42 Gt | Propylenglykol-monomethylether-acetat. |

Nach einer Belichtung mit Strahlung einer Wellenlänge von 260 nm und einer Energie von 55 bzw. 115 mJ/cm² erhielt man Strukturen, die keine praxisgerechte Bilddifferenzierung zeigten.

Bei Verwendung des Onium-Salzes (Beispiel 3) wurden auch in den unbelichteten Bereichen Lackreste beobachtet, d. h. Resistreste hafteten in den unbelichteten Bereichen am Substrat an, während bei Verwendung des Tosylesters (Beispiel 4) unterschnittene Resistprofile sichtbar waren, die auch bei verstärkter Belichtung nur auf Kosten der Wiedergabegenauigkeit beseitigt werden konnten. In beiden Fällen wurden somit keine akzeptablen Strukturierungen erhalten.

## Patentansprüche

1. Negativ arbeitendes strahlungsempfindliches Gemisch mit
a) einer Verbindung, die unter Einwirkung von aktinischer Strahlung eine starke Säure bildet,
b) einer Verbindung mit mindestens zwei durch Säure vernetzbaren Gruppen und
c) einem in Wasser unlöslichen, in wäßrig-alkalischen Lösungen löslichen oder zumindest quellbaren polymeren Bindemittel,
dadurch gekennzeichnet, daß die Verbindung (a) ein mit 2, 3 oder 4 Sulfonsäuren der allgemeinen Formel R-SO₃H verestertes Di-, Tri- bzw. Tetrahydroxybenzol, das ggf. noch mit einem Rest R' substituiert ist, darstellt, wobei R und R' gleiche oder verschiedene Bedeutung haben und R einen ggf. weiter substituierten (C₅-C₁₀)Cycloalkyl-, (C₆-C₁₀)Aryl-, (C₆-C₁₀)Aryl-(C₁-C₁₀)alkyl- oder (C₃-C₉)Heteroarylrest und R' einen der für R genannten Reste, einen (C₁-C₁₀)Alkyl-, (C₁-C₁₀)Alkanoyl-, (C₁-C₁₆)-Alkoxycarbonyl- oder einen (C₇-C₁₁)Aroylrest bezeichnet.

2. Negativ arbeitendes strahlungsempfindliches Gemisch gemäß Anspruch 1, dadurch gekennzeichnet, daß die Verbindung a) ein mit 2 oder 3 Sulfonsäuren R-SO₃H verestertes Di- bzw. Trihydroxybenzol, das gegebenenfalls noch mit einem Rest R' substituiert ist, darstellt.

3. Negativ arbeitendes strahlungsempfindliches Gemisch gemäß einem der Ansprüche 1 oder 2, dadurch gekennzeichnet, daß der Rest R substituiert ist mit mindestens einem Substituenten ausgewählt aus der Gruppe bestehend aus (C₁-c₈)Alkyl, (C₁-C₈)Alkoxy, (C₁-C₈)Alkanoyl, (C₁-C₈)Alkanoyloxy, (C₆-C₁₀)Aryl, Cyano oder Halogen.

4. Negativ arbeitendes strahlungsempfindliches Gemisch gemäß Anspruch 3, dadurch gekennzeichnet, daß die aromatischen Reste R' substituiert sind mit mindestens einem Substituenten ausgewählt aus der Gruppe bestehend aus (C₁-C₈) Alkyl, (C₁-C₈) Alkoxy, (C₁-C₈)-Alkanoyl, (C₁-C₈) Alkanoyloxy, Halogen und Sulfonyloxy der allgemeinen Formel -O-SO₂-R, in der R die im Anspruch 1 angegebene Bedeutung hat.

5. Negativ arbeitendes strahlungsempfindliches Gemisch gemäß einem oder mehreren der Ansprüchen 1 bis 4, dadurch gekennzeichnet, daß die Verbindung a) einen Anteil von 0,5 bis 25 Gew.-%, bevorzugt 3 -bis 15 Gew.-%, am Gesamtgewicht der Feststoffe des Gemisches hat.

6. Negativ arbeitendes strahlungsempfindliches Gemisch gemäß einem oder mehreren der Ansprüchen 1 bis 5, dadurch gekennzeichnet, das die Verbindung b) ein Resol ist.

7. Negativ arbeitendes strahlungsempfindliches Gemisch gemäß einem oder mehreren der Ansprüche 1 bis 5, dadurch gekennzeichnet, das die Verbindung b) ein mit Alkoxymethyl- oder Oxiranylmethyl-Gruppen substituierter Aromat ist.

8. Negativ arbeitendes strahlungsempfindliches Gemisch gemäß einem oder mehreren der Ansprüche 1 bis 5, dadurch gekennzeichnet, das die Verbindung b) ein Melamin/- oder Harnstoff/ Formaldehyd-Kondensat ist.

9. Negativ arbeitendes strahlungsempfindliches Gemisch gemäß einem oder mehreren der Ansprüche 1 bis 8, dadurch gekennzeichnet, daß die Verbindung mit mindestens zwei durch Säure vernetzbaren Gruppen b) einen Anteil von 1 bis 50 Gew.-%, bevorzugt 5 bis 25 Gew.-%, am Gesamtgewicht der Feststoffe des strahlungsempfindlichen Gemisches hat.

10. Negativ arbeitendes strahlungsempfindliches Gemisch gemäß einem oder mehreren der Ansprüche 1 bis 9, dadurch gekennzeichnet, daß das Bindemittel c) bis zu 30 Gew.-%, insbesondere bis zu 20 Gew.-%, eines Novolak-Kondensationsharzes enthält.

11. Negativ arbeitendes strahlungsempfindliches Gemisch gemäß einem oder mehreren der Ansprüche 1 bis 9, dadurch gekennzeichnet, daß das Bindemittel c) phenolische Hydroxygruppen enthält.

12. Negativ arbeitendes strahlungsempfindliches Gemisch gemäß einem oder mehreren der Ansprüche 1 bis 11, dadurch gekennzeichnet, daß das Bindemittel c) für Strahlung einer Wellenlänge 248 nm eine Extinktion von < 0,5 µm⁻¹, bevorzugt < 0,3 µm⁻¹, aufweist.

13. Negativ arbeitendes strahlungsempfindliches Gemisch gemäß einem oder mehreren der Ansprüche 1 bis 12, dadurch gekennzeichnet, daß das Bindemittel c) in einem Anteil von 40 bis 95 Gew.-%, bevorzugt 50 bis 90 Gew.-%, am Gesamtgewicht der Feststoffe des Gemisches hat.

14. Negativ arbeitendes strahlungsempfindliches Aufzeichungsmaterial, im wesentlichen bestehend aus einem Träger und einer strahlungsempfindlichen Schicht, dadurch gekennzeichnet, daß die Schicht ein strahlungshärtbares Gemisch gemäß einem oder mehreren der Ansprüche 1 bis 13 enthält.

## Claims

1. A negative-working radiation-sensitive mixture with
a) a compound which generates a strong acid under the action of actinic radiation,
b) a compound having at least two groups crosslinkable by acid and
c) a polymeric binder which is insoluble in water and soluble or at least swellable in an aqueous alkaline solution,
wherein the compound (a) is a di-, tri- or tetrahydroxybenzene which is esterified with respectively 2, 3 or 4 sulfonic acids of the formula R-SO₃H and may be further substituted by a radical R', where R and R' have identical or different definitions and R is a (C₅-C₁₀) cycloalkyl, (C₆-C₁₀)aryl, (C₆-C₁₀)aryl-(C₁-C₁₀)alkyl or (C₃-C₉)heteroaryl radical which may be further substituted, and R' is one of the radicals mentioned for R or is a (C₁-C₁₀)alkyl, (C₁-C₁₀) alkanoyl, (C₁-C₁₆) alkoxycarbonyl or a (C₇-C₁₁) aroyl radical.

2. A negative-working radiation-sensitive mixture as claimed in claim 1, wherein the compound a) is a di- or tri-hydroxybenzene which is esterified with respectively 2 or 3 sulfonic acids R-SO₃H and may be further substituted by a radical R'.

3. A negative-working radiation-sensitive mixture as claimed in either of claims 1 or 2, wherein the radical R is substituted by at least one substituent selected from the group comprising (C₁-C₈)alkyl, (C₁-C₈) alkoxy, (C₁-C₈) alkanoyl, (C₁-C₈) alkanoyloxy, (C₆-C₁₀)aryl, cyano or halogen.

4. A negative-working radiation-sensitive mixture as claimed in claim 3, wherein the aromatic radicals R' are substituted by at least one substituent selected from the group comprising (C₁-C₈)alkyl, (C₁-C₈) alkoxy, (C₁-C₈) alkanoyl, (C₁-C₈)alkanoyloxy, halogen and sulfonyloxy of the formula -O-SO₂-R, in which R is as defined in claim 1.

5. A negative-working radiation-sensitive mixture as claimed in one or more of claims 1 to 4, wherein the content of the compound a) in the total weight of the solids in the mixture is 0.5 to 25% by weight, preferably 3 to 15% by weight.

6. A negative-working radiation-sensitive mixture as claimed in one or more of claims 1 to 5, wherein the compound b) is a resol.

7. A negative-working radiation-sensitive mixture as claimed in one or more of claims 1 to 5, wherein the compound b) is an aromatic substituted by alkoxymethyl or oxiranylmethyl groups.

8. A negative-working radiation-sensitive mixture as claimed in one or more of claims 1 to 5, wherein the compound b) is a melamine/formaldehyde or urea/ formaldehyde condensate.

9. A negative-working radiation-sensitive mixture as claimed in one or more of claims 1 to 8, wherein the content of the compound having at least two groups crosslinkable by acid b) in the total weight of the solids in the radiation-sensitive mixture is 1 to 50% by weight, preferably 5 to 25% by weight.

10. A negative-working radiation-sensitive mixture as claimed in one or more of claims 1 to 9, wherein the binder c) contains up to 30% by weight, especially up to 20% by weight, of a novolak condensation resin.

11. A negative-working radiation-sensitive mixture as claimed in one or more of claims 1 to 9, wherein the binder c) contains phenolic hydroxy groups.

12. A negative-working radiation-sensitive mixture as claimed in one or more of claims 1 to 11, wherein the binder c) has an extinction of < 0.5 µm⁻¹, preferably < 0.3 µm⁻¹, for radiation of 248 nm wavelength.

13. A negative-working radiation-sensitive mixture as claimed in one or more of claims 1 to 12, wherein the content of the binder c) in the total weight of the solids in the mixture is 40 to 95% by weight, preferably 50 to 90% by weight.

14. A negative-working radiation-sensitive recording material, essentially composed of a support and a radiation-sensitive layer, wherein the layer contains a radiation-curable mixture as claimed in one or more of claims 1 to 13.

## Revendications

1. Mélange négatif sensible au rayonnement avec
a) un composé qui forme un acide fort sous l'effet d'un rayonnement actinique,
b) un composé avec au moins deux groupes réticulables par un acide et
c) un liant polymère insoluble dans l'eau, soluble ou au moins gonflable en solutions aqueuses alcalines,
caractérisé en ce que le composé (a) représente un di-, tri- ou tétrahydroxybenzène estérifié avec 2, 3 ou 4 acides sulfoniques de formule générale R-SO₃H, lequel est éventuellement substitué encore par un reste R', R et R' sont identiques ou différents et R représente un reste, éventuellement encore substitué, cycloalkyle en C₅-C₁₀, aryle en C₆-C₁₀, (aryle en C₆-C₁₀)-alkyle en C₁-C₁₀ ou hétéroaryle en C₃-C₉ et R' représente un des restes cités pour R, un reste alkyle en C₁-C₁₀, alkanoyle en C₁-C₁₀, alkoxycarbonyle en C₁-C₁₆ ou aroyle en C₇-C₁₁.

2. Mélange négatif sensible au rayonnement selon la revendication 1, caractérisé en ce que le composé a) représente un di-, respectivement trihydroxybenzène estérifié par 2 ou 3 acides sulfoniques R-SO₃H, lequel est éventuellement substitué encore par un reste R'.

3. Mélange négatif sensible au rayonnement selon la revendication 1 ou 2, caractérisé en ce que le reste R est substitué par au moins un substituant pris parmi les groupes alkyle en C₁-C₈, alkoxy en C₁-C₈, alcanoyle en C₁-C₈, alcanoyloxy en C₁-C₈, aryle en C₆-C₁₀, cyano ou halogène.

4. Mélange négatif sensible au rayonnement selon la revendication 3, caractérisé en ce que les restes aromatiques R' sont substitué par au moins un substituant pris parmi alkyle en C₁-C₈, alkoxy en C₁-C₈, alcanoyle en C1-C8, alcanoyloxy en C₁-C₈, halogène et sulfonyloxy de formule générale -O-SO₂-R, dans laquelle R a la signification donnée dans la revendication 1.

5. Mélange négatif sensible au rayonnement selon une des revendications 1 à 4, caractérisé en ce que le composé a) est contenu dans un taux de 0,5 à 25 % en poids, de préférence de 3 à 15 % en poids par rapport au poids total de l'extrait sec dans le mélange.

6. Mélange négatif sensible au rayonnement selon une ou plusieurs des revendications 1 à 5, caractérisé en ce que le composé b) est un résol.

7. Mélange négatif sensible au rayonnement selon une ou plusieurs des revendications 1 à 5, caractérisé en ce que le composé b) est un composé aromatique substitué par des groupes alkoxyméthyl ou oxirannyle.

8. Mélange négatif sensible au rayonnement selon une ou plusieurs des revendications 1 à 5, caractérisé en ce que le composé b) est un condensat de mélamine/formaldéhyde ou d'urée/formaldéhyde.

9. Mélange négatif sensible au rayonnement selon une ou plusieurs des revendications 1 à 8, caractérisé en ce que le composé avec au moins deux groupes b) réticulables par un acide présente une teneur de 1 à 50 % en poids, de préférence de 5 à 25 % en poids par rapport au poids total de l'extrait sec du mélange sensible au rayonnement conforme à l'invention.

10. Mélange négatif sensible au rayonnement selon une ou plusieurs des revendications 1 à 9, caractérisé en ce que le liant c) a une teneur jusqu'à 30 % en poids, en particulier jusqu'à 20 % en poids en une résine de condensation de novolaque.

11. Mélange négatif sensible au rayonnement selon une ou plusieurs des revendications 1 à 9, caractérisé en ce que le liant c) contient des groupes hydroxy phénoliques.

12. Mélange négatif sensible au rayonnement selon une ou plusieurs des revendications 1 à 11, caractérisé en ce que le liant c) présente, pour le rayonnement d'une longueur d'onde de 248 nm, une extinction de <0,5 µm-¹, de préférence <0,3 µm-¹.

13. Mélange négatif sensible au rayonnement selon une ou plusieurs des revendications 1 à 12, caractérisé en ce que le liant c) est présent dans un taux de 40 à 95 % en poids, de préférence de 50 à 90 % en poids, par rapport au poids total de l'extrait sec du mélange.

14. Matière d'enregistrement négative sensible au rayonnement constituée essentiellement d'un support et d'une couche sensible au rayonnement, caractérisée en ce que la couche contient un mélange photodurcissable selon une ou plusieurs des revendications 1 à 13.
